# EUROPEAN PATENT SPECIFICATION

(11) **EP 4 129 385 B1**
(45) Date of publication and mention of the grant of the patent: **10.09.2025**
(21) Application number: 21818435.6
(22) Date of filing: 27.05.2021
(51) Int. Cl.: A61M 25/09, A61B 5/0215, A61B 5/00, A61M 25/00

(54) **GUIDE WIRE**
FÜHRUNGSDRAHT
FIL GUIDE

(30) Priority: 02.06.2020 JP 2020095911
(43) Date of publication of application: 08.02.2023
(73) Proprietor: Nipro Corporation, Osaka-shi, Osaka 531-8510 (JP)
(72) Inventor: NARUSE, Natsumi, Osaka-shi, Osaka 531-8510 (JP); UEHARA, Yohei, Osaka-shi, Osaka 531-8510 (JP); YASUMURA Naoaki, Osaka-shi, Osaka 531-8510 (JP)
(74) Representative: Kraus & Lederer PartGmbB
(86) International application number: PCT/JP2021/020235
(87) International publication number: WO 2021/246287

(56) References cited:
- CN-A- 102 641 153
- JP-A- 2009 240 710
- JP-A- 2011 188 913
- JP-A- 2014 504 166
- US-A1- 2013 331 820
- US-A1- 2016 262 698
- US-A1- 2016 310 079
- US-A1- 2020 085 307

## Description

### TECHNICAL FIELD

The present invention relates to a guide wire having a sensor and inserted into a blood vessel.

### BACKGROUND ART

In order to detect various physical quantities in a blood vessel such as a blood pressure or a blood flow rate, inserting a guide wire having a sensor into the blood vessel is performed. The guide wire is inserted into a vein from a lower part of a clavicle or a femoral area, for example, and a tip end thereof is delivered to a coronary artery. Then, the blood pressure or the like at the coronary artery is measured by the sensor provided at the tip end of the guide wire (Patent Document 1).

The sensor is located in an internal space of a circular tube-shaped housing constituting a part of the guide wire. For example, a housing made of metal is suitable for protecting the sensor because rigidity is high, but is hard to bow along a curve of the blood vessel. As a result, there is a problem that it is hard to pass the housing through a curved portion of the blood vessel. For this problem, each of Patent Documents 2 and 3 discloses a configuration in which a slit is formed on the housing to make the housing easy to be curved.

### PRIOR ART DOCUMENTS

### PATENT DOCUMENTS

Patent Document 1: Japanese Patent Application Laid-Open No. 2003-225312
Patent Document 2: Japanese Patent Application Laid-Open No. 2014-147459
Patent Document 3: Japanese Patent No. 6395826 Furthermore reference is made to CN 102 641 153 A, US 2013/331820 A1, and US 2020/085307 A1.

### SUMMARY OF THE INVENTION

The invention is defined by the independent claim 1. Although the housing on which the slit is formed is easy to be curved, when the housing is curved, the slit is deformed and an end portion of the slit is easy to fracture. Furthermore, also when a tensile force acts on the housing, the end portion of the slit is easy to fracture. On the other hand, as disclosed in Patent Documents 2 and 3, when the length of the slit in an extending direction is relatively shortened, strength of the slit increases, but the housing becomes hard to be curved or hard to be extended, and operability is impaired.

The present invention has been made in view of the above-described circumstances, and an object thereof is to provide a guide wire in which a housing of a sensor is easy to be curved.

### MEANS FOR SOLVING THE PROBLEMS

(1) A guide wire is provided as mentioned in claim 1 and, according to the present invention inter alia includes a wire material, a circular tube-shaped housing attached to the wire material, and a sensor located in an internal space of the housing. The housing has a slit penetrating a peripheral wall of the housing and extending in a helix. The slit has a central portion extending in the helix in a constant extending direction along the peripheral wall, and an end portion including one end of the slit and bent with respect to the central portion along a bending direction, the bending direction intersecting the extending direction.
   By the slit, the housing becomes easy to be curved and easy to be extended. By the end portion of the slit, even when a tensile force acts on the housing, the end portion is hard to fracture.
(2) Preferably, the bending direction is parallel with an axis of the housing.
(3) Preferably, the end portion is curved in a U-shape.
(4) Preferably, a bending point of the central portion and the end portion has a round shape.
(5) Preferably, the pitch of the helix in the central portion of the first slit is larger in both end sides than at a center.

In the central portion of the first slit, the both end sides become easy to withstand pulling, and the center becomes easy to be extended in an axial direction.

According to the invention, the housing further has a second slit penetrating the peripheral wall of the housing and extending in a helix, and the first slit and the second slit form a double helix located alternately with respect to an axial direction of the housing.

By the double helix, the housing becomes further easy to be curved.

(6) Preferably, a synthetic resin is filled in the internal space of the housing, the internal space surrounded by the peripheral wall on which the slit is located.

Since a tensile force acting on the housing also acts on the synthetic resin, tensile strength is improved as a whole of the housing and the synthetic resin.

### EFFECTS OF THE INVENTION

According to the present invention, the housing of the sensor is easy to be curved, and the slit is hard to fracture.

### BRIEF DESCRIPTION OF THE DRAWINGS

Fig. 1 is a schematic diagram of a guide wire system 10.
Fig. 2 is a diagram showing a guide wire 30.
Fig. 3 is a perspective view of a pressure sensor 11.
Fig. 4 is a cross-sectional view showing an internal configuration of a housing 34.
Fig. 5 is a diagram showing slits 51 to 54.
Fig. 6 is a partially enlarged view of Fig. 5.
Fig. 7 is an enlarged view showing the slit 51 according to a modification example.
Fig. 8 is an enlarged view showing the slit 51 according to a modification example.
Fig. 9 is a diagram showing the housing 34 according to a comparative example.
Fig. 10 is a diagram showing the housing 34 according to a comparative example.

### MODES FOR CARRYING OUT THE INVENTION

Hereinafter, preferred embodiments of the present invention are described. Note that it is needless to say that each embodiment is merely one embodiment of the present invention and that the embodiment can be changed without departing from the gist of the present invention.

### [Guide wire system 10]

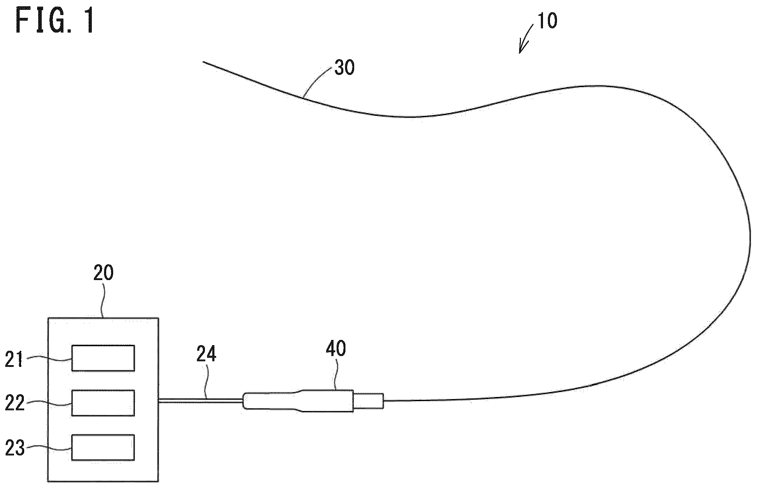

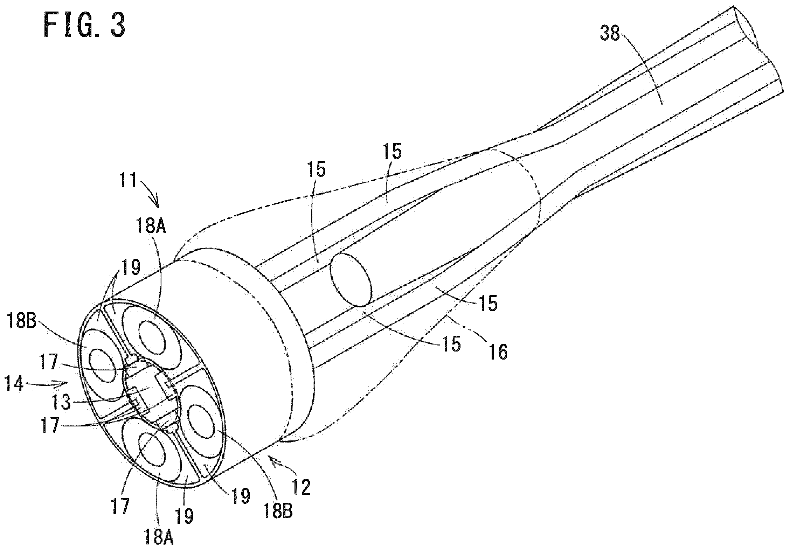

As shown in Fig. 1, a guide wire system 10 includes a guide wire 30, an arithmetic device 20, and a female-type connector 40 connecting the guide wire 30 and the arithmetic device 20. The guide wire 30 is an elongated rope body and is insertable into a blood vessel such as a coronary artery. The guide wire 30 includes, at a distal end portion, a pressure sensor 11 (see Fig. 3, an example of a sensor) that outputs electrical information in accordance with a pressure in the blood vessel.

The arithmetic device 20 includes a power supply unit 21 that supplies current to the pressure sensor 11 of the guide wire 30, an arithmetic unit 22 that performs arithmetic processing on the electrical information output from the pressure sensor 11, and a memory 23 that stores information necessary for the arithmetic processing. The electrical information output from the pressure sensor 11 is transmitted from the guide wire 30 via the female-type connector 40 and a cable 24 to the arithmetic unit 22. The arithmetic unit 22 calculates a blood pressure based on the electrical information output from the pressure sensor 11. In short, the guide wire system 10 is used to measure the blood pressure.

In Fig. 1, of both ends of the guide wire 30, a fixed end (end connected to the female-type connector 40) is a proximal end (lower left end in Fig. 1), and a free end (tip end when inserted into the blood vessel) is a distal end (upper left end in Fig. 1). In the present specification, in the guide wire 30, a side on which there is the proximal end is referred to as a proximal end side, and a side on which there is the distal end is referred to as a distal end side.

### [Guide wire 30]

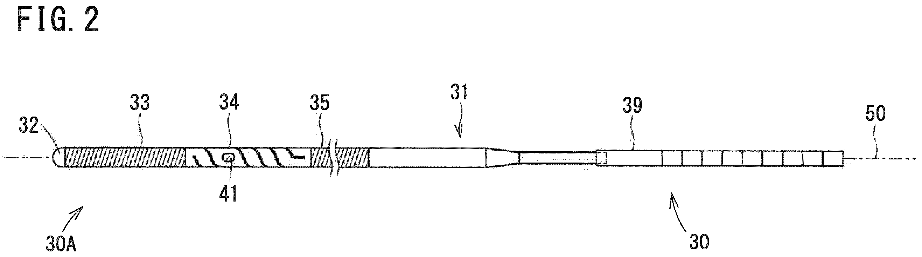

The guide wire 30 is shown in Fig. 2. In Fig. 2, the left side is the distal end side of the guide wire 30, and the right side is the proximal end side of the guide wire 30. The guide wire 30 is roughly divided into a tip end portion 30A (an example of a distal end portion), a core wire 31 (an example of a main body), and a male-type connector 39 (an example of a connector). The tip end portion 30A includes a tip end guide portion 32, a first helical body 33, the housing 34, and a second helical body 35. Note that an axis 50 indicates an axis of the guide wire 30 when the guide wire 30 is in a straight state without being bowed or curved.

The core wire 31 is a columnar shaped-member and is a solid material made of stainless steel, for example. The tip end guide portion 32 is a hemispherical shaped-member disposed at the distal end and protruding to the distal end side, and abuts a blood vessel wall, thereby guiding a traveling direction of the guide wire 30 so as to follow the blood vessel. The first helical body 33 and the second helical body 35 are wire materials wound in a helical shape, and are configured to be bent more easily than the core wire 31 so that the distal end portion of the guide wire 30 is easy to follow the blood vessel.

The housing 34 is a casing that accommodates the pressure sensor 11 in an internal space thereof. The housing 34 has a circular tube shape. The housing 34 has two through holes 41. Note that the two through holes 41 are disposed symmetrically by 180° with respect to the axis 50, and only one of the through holes 41 appears in Fig. 2. Blood enters an inside of the housing 34 via the through holes 41 and contacts a diaphragm 13 (Fig. 3) of the pressure sensor 11. The housing 34 has slits 51, 52, 53, 54. The slits 51, 52, 53, 54 will be described later in detail.

A taper pin 38 (see Fig. 3) extends from the distal end of the core wire 31 toward the housing 34 in an internal space of the second helical body 35. The taper pin 38 is a member that reinforces bending rigidity of the second helical body 35. The taper pin 38 has a columnar shape, and its outer diameter gradually decreases from the distal end of the core wire 31 toward the housing 34. Note that although not shown in each drawing, a tip end guide pin extends from the distal end of the housing 34 toward the tip end guide portion 32 in an internal space of the first helical body 33. The tip end guide pin has a columnar shape and is a member that reinforces bending rigidity of the first helical body 33. The tip end guide pin is fixed to the housing 34 and the tip end guide portion 32. The male-type connector 39 is provided at the proximal end of the core wire 31. The male-type connector 39 is inserted into the female-type connector 40, thereby the pressure sensor 11 and the arithmetic device 20 are electrically connected. The core wire 31, the first helical body 33, and the second helical body 35 are examples of a wire material.

As shown in Fig. 3, the pressure sensor 11 includes a sensor main body 12, the diaphragm 13, a bridge circuit 14, four conductive wires 15, and a connecting portion 16. The sensor main body 12 is fixed to the taper pin 38 fixed to the core wire 31, by the connecting portion 16 configured by an adhesive, for example. The diaphragm 13, the bridge circuit 14, and the four conductive wires 15 are attached to the sensor main body 12. The bridge circuit 14 is a full-bridge circuit in which all of four resistive bodies 17 function as strain gauges for measurement. The bridge circuit 14 includes the four resistive bodies 17, four terminals 18A, 18B, and four connecting bodies 19. The four resistive bodies 17 are fixed to the diaphragm 13. The four terminals 18A, 18B consist of two input terminals 18A and two output terminals 18B. Each connecting body 19 electrically connects each resistive body 17 to each of the terminals 18A, 18B. Each conductive wire 15 is electrically connected to each of the terminals 18A, 18B, extends toward a base end in an internal space of the core wire 31, and is electrically connected to each connecting terminal of the male-type connector 39.

In a state in which the guide wire 30 is inserted into the blood vessel and the blood pressure is applied to the pressure sensor 11, the diaphragm 13 is elastically deformed in accordance with the blood pressure. Along with the elastic deformation of the diaphragm 13, the four resistive bodies 17 are elastically deformed, and electric resistance values of the four resistive bodies 17 are changed. When a voltage is applied between the two input terminals 18A in this state, a potential difference is generated between the two output terminals 18B. Based on the potential difference, the blood pressure is calculated in the arithmetic device 20 (Fig. 1).

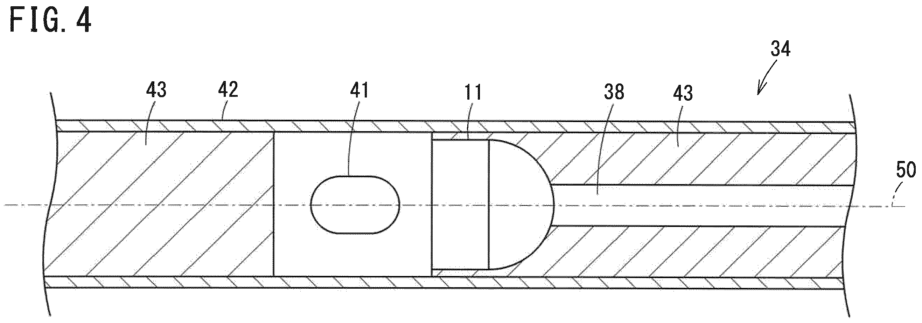

As shown in Fig. 4, the pressure sensor 11 is located on the proximal end side of the through hole 41 in the internal space of the housing 34. A synthetic resin 43 is filled in the internal space of the housing 34, the internal space located on the proximal end side of the sensor main body 12 of the pressure sensor 11. Furthermore, the synthetic resin 43 is also filled in the internal space of the housing 34, the internal space located on the distal end side of the through hole 41. The slits 51 to 54 to be described later are located on a peripheral wall 42 of the housing 34, the peripheral wall 42 partitioning the internal space filled with the synthetic resin 43. The synthetic resin 43 is an epoxy resin, a urethane resin, or a polyamide elastomer resin, for example.

### [Slits 51, 52]

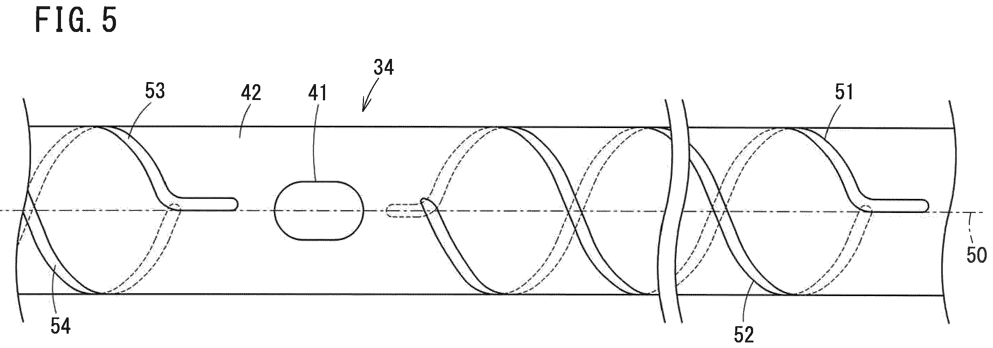

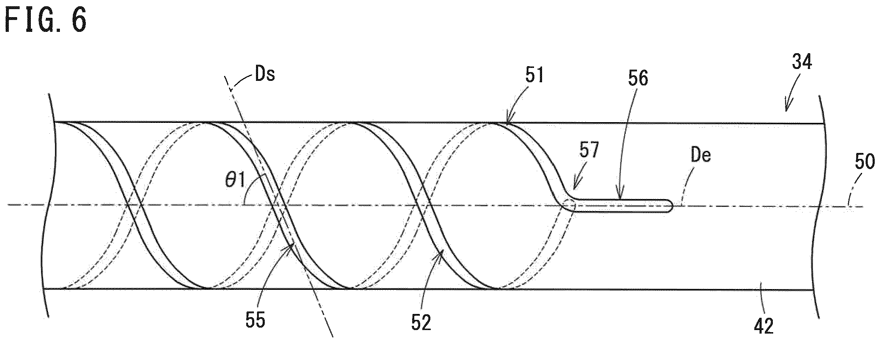

As shown in Fig. 5, the slits 51 to 54 are formed on the housing 34. Each of the slits 51 to 54 penetrates the peripheral wall 42 of the housing 34. The slits 51 to 54 extend in helices, taking the axis 50 of the housing 34 as a center. As shown in Fig. 6, when the housing 34 is viewed from a direction orthogonal to the axis 50, an included angle θ1 formed by intersecting an extending direction Ds in which each of the slits 51 to 54 extends and the axis 50 is constant, and is approximately 60° in the present embodiment. Note that the extending directions Ds in which the slits 51 to 54 extend are parallel. In the present embodiment, when the housing 34 is viewed from the proximal end to the distal end along the axis 50, the slits 51 to 54 each extend toward the distal end while rotating clockwise.

As shown in Fig. 5, the slits 51, 52 are located on a proximal side of the through hole 41, in the housing 34. The slits 53, 54 are located on a distal side of the through hole 41, in the housing 34. The slits 51, 52 form a double helix located alternately with respect to the axis 50 of the housing 34. In other words, the slits 51, 52 are shifted in phase by a half cycle around the axis 50. In further other words, the slit 52 is located at a position shifted by a half of a distance traveled by the slit 51 along the axis 50 in a cycle in which the slit 51 rotates one time around the axis 50. The slits 53, 54 form a double helix as with the slits 51, 52.

As shown in Fig. 6, the slit 51 has a central portion 55 forming a constant included angle θ1 with respect to the axis 50 and end portions 56 located on both sides of the axis 50 with respect to the central portion 55. In the present embodiment, since the two end portions 56 are located symmetrically by 180° with respect to the axis 50, the end portion 56 located on the distal end side is shown by a broken line in Fig. 5. Since the two end portions 56 has the same positional relationship with respect to the central portion 55 except that the positions with respect to the axis 50 and the extending directions are symmetric, hereinafter, the end portion 56 located on the proximal end side will be described in detail as an example.

As shown in Fig. 6, the central portion 55 extends forming a helix in which the extending direction Ds is constant. The end portion 56 is continuous with the proximal end of the central portion 55. The end portion 56 constitutes one end of the slit 51. Most part of the end portion 56 follows a bending direction De intersecting the extending direction Ds. In the present embodiment, the bending direction De is parallel with the axis 50. A connecting point 57 of the central portion 55 and the end portion 56 forms a round shape bending smoothly. Most part of the end portion 56, the part extending to the proximal end has a linear shape, and the linear-shaped portion follows the bending direction De. In the proximal end of the slit 51, a pitch of the helix increases toward the proximal end, by the end portion 56.

Although detailed description is omitted, the slit 53 has a central portion and end portions similar to those of the slit 51. Furthermore, in the present embodiment, the slits 52, 54 do not have, on both ends, the end portions 56 such as the slit 51 has, and extend along the extending direction Ds over the entire range.

### [Actions and effects of the present embodiment]

According to the guide wire 30 according to the above-described embodiment, since the slits 51 to 54 are formed on the housing 34, the housing 34 becomes easy to be curved and becomes easy to be extended along the axis 50. Furthermore, since the formed slit 51 has the central portion 55 and the end portions 56, even if a tensile force along the axis 50 acts on the housing 34, the end portions 56 are hard to fracture.

Furthermore, since the synthetic resin 43 is filled in the internal space of the housing 34, the space surrounded by the peripheral wall 42 on which the slits 51 to 54 are located, a tensile force acting along the axis 50 of the housing 34 also acts on the synthetic resin 43, and tensile strength is improved as a whole of the housing 34 and the synthetic resin 43.

### [Modification and comparative examples]

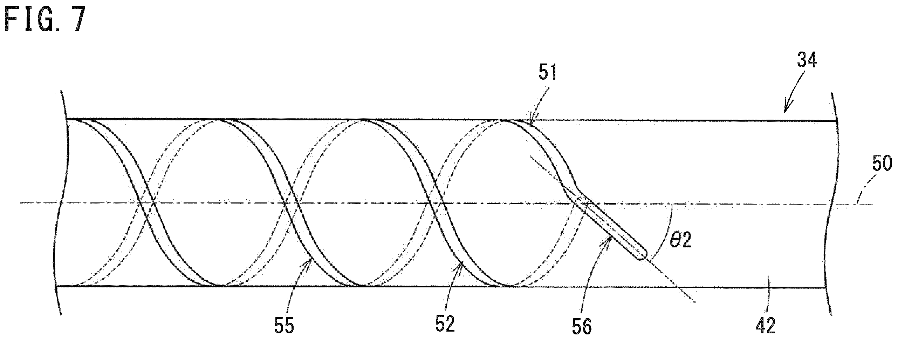

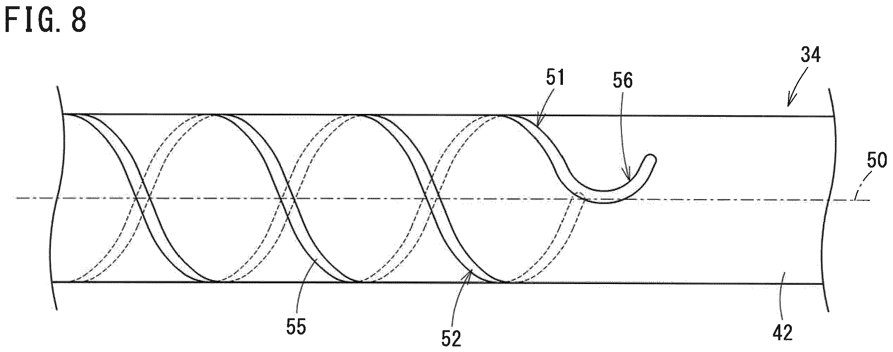

Although the bending direction De along which the end portion 56 extends follows the axis 50 in the above-described embodiment, the bending direction De may not necessarily follow the axis 50. For example, as shown in Fig. 7, an included angle θ2 formed by the end portion 56 and the axis 50 may be 15°, 30°, 45°, or the like. Furthermore, as shown in Fig. 8, the end portion 56 may be curved in a U-shape from the central portion 55 and may extend in a so-called opposite direction.

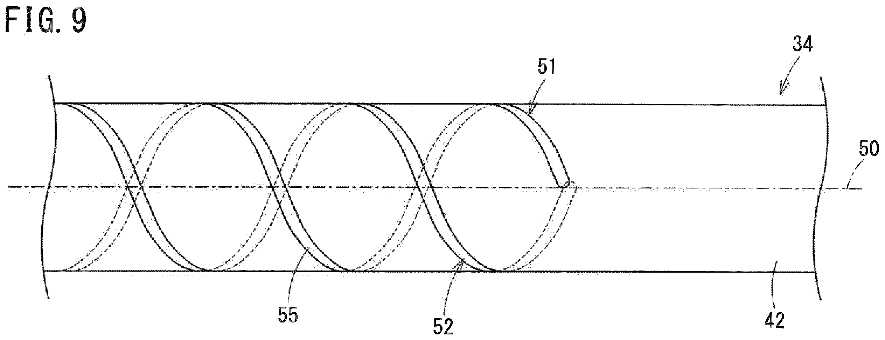

Furthermore, although the slits 51, 53 have the end portions 56 in the above-described embodiment, the slits 51, 53 may not necessarily have the end portions 56 and may only have the central portion 55, as shown in the comparative example of Fig. 9. In this aspect, tensile strength of the housing 34 is improved by the synthetic resin 43 filled in the internal space of the housing 34, the internal space surrounded by the peripheral wall 42 on which the slits 51 to 54 are located.

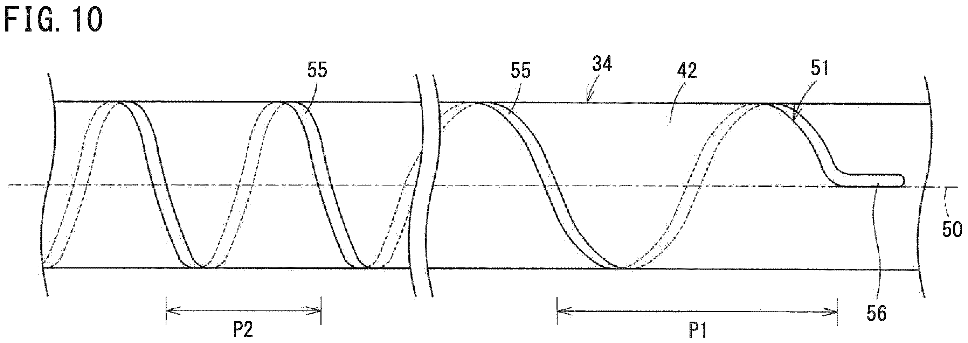

Furthermore, although the slits 51, 52 and the slits 53, 54 form the double helix in the above-described embodiment, the slit 51 and the slit 53 may be formed on the housing 34 as a single helix, without being provided with the slit 52 and the slit 54. Furthermore, the pitch of the slits 51 may not necessarily be constant. For example, as shown in the comparative example of Fig. 10, the slit 51 may be formed on the housing 34 as the single helix, and regarding a pitch (distance along the axis 50 between adjacent slits 51) of the helix in the central portion 55 of the slit 51, a pitch P1 in both end sides may be larger than a pitch P2 at a center (P1 > P2).

As the pitch of the helix of the slit 51 becomes smaller, the housing 34 becomes easy to be extended along the axis 50, whereas as the pitch becomes larger, tensile strength along the axis 50 becomes larger. When a tensile force along the axis 50 acts on the housing 34, in the central portion 55 of the slit 51, the center having the smaller pitch (P2) is extended more than the both ends having the larger pitch (P1). The central portion 55 of the slit 51 is extended along the axis 50, thereby tensile length (stroke) until the housing 34 fractures becomes longer.

When the central portion 55 of the slit 51 is fully extended both at the center and in the both ends and tensile strength of the both ends of the central portion 55 is eventually exceeded, the housing 34 fractures near a boundary of the central portion 55 and the end portion 56 (near both ends of the central portion 55). Therefore, by decreasing the pitch P2 at the center of the central portion 55 of the slit 51, the slit 51 can be made to be easy to extended along the axis 50 and stroke needed until the housing 34 fractures can be increased, whereas, by increasing the pitch P1 at the both ends of the central portion 55 of the slit 51, tensile force capable of withstanding until the fracture can be increased.

Furthermore, the pressure sensor 11 provided to the guide wire 30 is merely an example of a sensor, and other sensors or electronic circuits that measure physical quantities (temperature, flow velocity, or the like) of blood or the blood vessel other than the pressure may be provided. Furthermore, it is needless to say that the configuration of the distal end side of the guide wire 30 shown in the above-described embodiment is merely an example, and that the configurations of the helical body, the taper pin, the housing, or the like may be changed appropriately.

### EXAMPLES

### [Examples 1 to 5]

A circular tube made of stainless steel (SUS304) having a length of 7 mm, an outer diameter of 0.37 mm, and a thickness of 0.03 mm was used as the housing, the width of the slit was set to 0.02 mm, the included angle θ1 formed by the axis and the slit on a single helix was set to 60°, guide wires in which the included angle θ2 formed by the axis and an extending direction of the end portion of the slit was set to 15°, 30°, and 45°, a guide wire in which the end portion is parallel with the axis, and a guide wire in which the end portion is curved in a U-shape (see Fig. 8) were formed, and these were respectively named Examples 1 to 5.

### [Examples 6 to 8]

A circular tube made of stainless steel (SUS304) having a length of 7 mm, an outer diameter of 0.37 mm, and a thickness of 0.03 mm was used as the housing, the width of the slit was set to 0.02 mm, the axis and the end portion of the slit on a single helix were extended in parallel with the axis, guide wires in which a radius R at a boundary of the central portion and the end portion was set to 0.05, 0.3, and 0.4 were formed, and these were respectively named Examples 6 to 8.

### [Further Comparative example]

A circular tube made of stainless steel (SUS304) having a length of 7 mm, an outer diameter of 0.37 mm, and a thickness of 0.03 mm was used as the housing, the width of the slit was set to 0.02 mm, a guide wire having the axis and the slit on a single helix, the slit not having the end portion was formed, and this was named a comparative example.

### [Tensile strength]

In a state in which a wire material having a diameter of 0.08 mm was inserted into the housing for stabilizing the shape of a slit helical portion at the time of pulling, tensile strength when one end of the housing of each example and the comparative example was fixed and the other end was pulled was obtained using a simulation software. As for material properties of SUS304, Young's modulus was set to 200 GPa, Poisson's ratio was set to 0.3, yield stress was set to 250 MPa, and tangent modulus was set to 1,450 MPa. The results are shown in Table 1.

**[Table 1]**

| | Example 1 | Example 2 | Example 3 | Example 4 | Example 5 |
|---|---|---|---|---|---|
| | *θ*2=15° | *θ*2=30° | *θ*2=45° | Parallel with axis | Curved in U-shape |
| Tensile strength (N) | 0.76 | 1.09 | 1.48 | 1.78 | 1.72 |

| | Example 6 | Example 7 | Example 8 | Comparable example |
|---|---|---|---|---|
| | R = 0.05 | R=0.3 | R=0.4 | |
| Tensile strength (N) | 1.15 | 1.72 | 1.99 | 0.37 |

As is clear from Table 1, in all of Examples 1 to 8, tensile strength was improved compared with the comparative example. Among Examples 1 to 5, as the included angle θ1 became larger, the tensile strength became stronger. Furthermore, Example 4 in which the extending direction of the end portion of the slit was parallel with the axis represented the strongest result. Among Examples 6 to 8, as the radius R became larger, the tensile strength became stronger.

### [Examples 9 to 12]

A circular tube made of stainless steel (SUS304) having a length of 7 mm, an outer diameter of 0.37 mm, and a thickness of 0.03 mm was used as the housing, the width of the slit was set to 0.02 mm, the axis and the end portion of the slit on a single helix extends in parallel with the axis of the housing, guide wires in which the pitch (length between adjacent slits along the axial direction of the circular tube) of the slit on the single helix of 7 mm in total length was set to 100 µm, 150 µm, 200 µm, and 280 µm were formed, and these were named Examples 9 to 12.

A tensile strength test was performed on Examples 9 to 12 using the simulation software to which the same setting as the above-described one has been performed. Stroke length (mm) and tensile strength (N) at the time of the fracture are shown in Table 2. Note that in Example 9, even when the stroke length became 15 mm, a maximum equivalent stress needed for the fracture was not reached.

**[Table 2]**

| | Example 9 | Example 10 | Example 11 | Example 12 |
|---|---|---|---|---|
| Helical pitch | 100*µ*m | 150*µ*m | 200*µ*m | 280*µ*m |
| Stroke (mm) | >15 | 9.5 | 6.5 | 3.8 |
| Tensile strength (N) | - | 1.6 | 1.8 | 2.3 |

As is clear from Table 2, as the pitch of the slits became larger, the stroke length became shorter and the tensile strength became larger. From this, it can be said that when the pitch of the slit increases, although the slit portion becomes hard to be extended, the tensile strength becomes stronger.

### DESCRIPTION OF REFERENCE CHARACTERS

- 11: pressure sensor (sensor)
- 30: guide wire
- 31: core wire (wire material)
- 33: first helical body (wire material)
- 34: housing
- 35: second helical body (wire material)
- 42: peripheral wall
- 43: synthetic resin
- 51 to 54: slit
- 55: central portion
- 56: end portion

## Claims

1. A guide wire (30) comprising:
a wire material;
a circular tube-shaped housing (34) attached to the wire material; and
a sensor (11) located in an internal space of the housing,
wherein
the housing (34) has a first slit penetrating a peripheral wall of the housing and extending in a helix, and
the first slit has:
a central portion (55) extending in the helix in a constant extending direction along the peripheral wall; and
an end portion (56) including one end of the first slit and bent with respect to the central portion along a bending direction, the bending direction intersecting the extending direction, wherein
the housing (34) further has a second slit penetrating the peripheral wall of the housing and extending in a helix, and
the first slit and the second slit form a double helix located alternately with respect to an axial direction of the housing.

2. The guide wire according to claim 1, wherein the bending direction is parallel with an axis of the housing (34).

3. The guide wire according to claim 1, wherein the end portion (56) is curved in a U-shape.

4. The guide wire according to any one of claims 1 to 3, wherein a bending point of the central portion (55) and the end portion (56) has a round shape.

5. The guide wire according to any one of claims 1 to 4, wherein the pitch of the helix in the central portion (55) of the first slit is larger in both end sides than at a center.

6. The guide wire according to any one of claims 1 to 5, wherein a synthetic resin (43) is filled in the internal space of the housing, the internal space surrounded by the peripheral wall on which the slit is located.

## Patentansprüche

1. Führungsdraht (30), umfassend:
ein Drahtmaterial;
ein kreisförmiges rohrförmiges Gehäuse (34), das an dem Drahtmaterial befestigt ist; und
einen Sensor (11), der in einem Innenraum des Gehäuses angeordnet ist, wobei
das Gehäuse (34) einen ersten Schlitz aufweist, der eine Umfangswand des Gehäuses durchdringt und sich in einer Helix erstreckt, und
wobei der erste Schlitz Folgendes aufweist:
einen Mittelabschnitt (55), der sich in der Helix in einer konstanten Erstreckungsrichtung entlang der Umfangswand erstreckt; und
einen Endabschnitt (56), der ein Ende des ersten Schlitzes beinhaltet und in Bezug auf den Mittelabschnitt entlang einer Biegerichtung gebogen ist, wobei die Biegerichtung die Erstreckungsrichtung schneidet, wobei
das Gehäuse (34) ferner einen zweiten Schlitz aufweist, der die Umfangswand des Gehäuses durchdringt und sich in einer Helix erstreckt, und
der erste Schlitz und der zweite Schlitz eine Doppelhelix bilden, die abwechselnd in Bezug auf eine axiale Richtung des Gehäuses angeordnet ist.

2. Führungsdraht nach Anspruch 1, wobei die Biegerichtung parallel zu einer Achse des Gehäuses (34) verläuft.

3. Führungsdraht nach Anspruch 1, wobei der Endabschnitt (56) in einer U-Form gekrümmt ist.

4. Führungsdraht nach einem der Ansprüche 1 bis 3, wobei ein Biegepunkt des Mittelabschnitts (55) und des Endabschnitts (56) eine runde Form aufweist.

5. Führungsdraht nach einem der Ansprüche 1 bis 4, wobei die Steigung der Helix in dem Mittelabschnitt (55) des ersten Schlitzes an beiden Endseiten größer ist als an einer Mitte.

6. Führungsdraht nach einem der Ansprüche 1 bis 5, wobei ein synthetisches Kunstharz (43) in den Innenraum des Gehäuses gefüllt ist, wobei der Innenraum von der Umfangswand umgeben ist, an der der Schlitz angeordnet ist.

## Revendications

1. Fil guide (30) comprenant :
un matériau de fil ;
un boîtier en forme de tube circulaire (34) fixé au matériau de fil ; et
un capteur (11) situé dans un espace interne du boîtier, dans lequel
le boîtier (34) présente une première fente pénétrant une paroi périphérique du boîtier et se prolongeant en hélice, et
la première fente présente :
une partie centrale (55) se prolongeant en ladite hélice dans une direction d'extension constante le long de la paroi périphérique ; et
une partie d'extrémité (56) comprenant une extrémité de la première fente et courbée par rapport à la partie centrale le long d'une direction de courbure, la direction de courbure coupant la direction d'extension, dans lequel
le boîtier (34) présente en outre une seconde fente pénétrant la paroi périphérique du boîtier et se prolongeant en hélice, et
la première fente et la seconde fente forment une double hélice située alternivement d'un côté et de l'autre par rapport à une direction axiale du boîtier.

2. Fil guide selon la revendication 1, dans lequel la direction de courbure est parallèle à un axe du boîtier (34).

3. Fil guide selon la revendication 1, dans lequel la partie d'extrémité (56) est courbée en forme de U.

4. Fil guide selon l'une quelconque des revendications 1 à 3, dans lequel un point de courbure de la partie centrale (55) et de la partie d'extrémité (56) a une forme arrondie.

5. Fil guide selon l'une quelconque des revendications 1 à 4, dans lequel le pas de l'hélice dans la partie centrale (55) de la première fente est plus élevé au niveau des deux côtés d'extrémité qu'au centre.

6. Fil guide selon l'une quelconque des revendications 1 à 5, dans lequel une résine synthétique (43) est introduite dans l'espace interne du boîtier, l'espace interne étant entouré par la paroi périphérique dans laquelle est située la fente.
